# EUROPEAN PATENT SPECIFICATION

(11) **EP 3 145 483 B1**
(45) Date of publication and mention of the grant of the patent: **25.04.2018**
(21) Application number: 15729943.9
(22) Date of filing: 17.03.2015
(51) Int. Cl.: A61K 8/46, A61Q 5/02

(54) **FOAMING COMPOSITION CONTAINING INTERNAL OLEFIN SULFONATES AND ONE POLYMERIC SUSPENSION AGENT**
SCHÄUMENDE ZUSAMMENSETZUNG MIT INTERNEN OLEFIN-SULFONATEN UND EINEM POLYMERSUSPENSIONSMITTEL
COMPOSITION MOUSSANTE CONTENANT DES SULFONATES D'OLÉFINE INTERNE ET UN AGENT STABILISATEUR POLYMÈRE

(30) Priority: 19.03.2014 JP 2014056154
(43) Date of publication of application: 29.03.2017
(73) Proprietor: L'OREAL, 75008 Paris (FR); Ji, Jinglan, Kawagawa 2130012 (JP); Pistorio, Bradford, Kawagawa 2130012 (JP)
(72) Inventor: LI Jinglan, Kawasaki-shi Kawagawa 2130012 (JP); PISTORIO Bradford, Kawasaki-shi Kawagawa 2130012 (JP)
(74) Representative: Casalonga
(86) International application number: PCT/JP2015/058648
(87) International publication number: WO 2015/141863

(56) References cited:
- WO-A2-2014/046303
- DE-A1- 19 845 456
- US-A- 3 332 880
- US-A- 4 852 653
- US-A- 5 580 494

## Description

### TECHNICAL FIELD

The present invention relates to a composition containing, in an aqueous medium, at least:
a) a specific sulfonate mixture which will be defined in greater detail hereinbelow and
b) at least one polymeric suspension agent.

The present invention relates also to a process for cleansing keratin materials, which consists of applying to the said keratin materials a composition according to the invention, in working the said composition into a foam and then in rinsing off the said composition, especially with water.

The present invention relates also to the cosmetic use of the composition as defined above, for removing makeup and/or cleansing the skin, the hair and/or mucous membranes, or for skincare.

### BACKGROUND ART

Cleansing the skin is very important for caring for the face. It must be as efficient as possible because greasy residues, such as excess sebum, the remnants of cosmetic products used daily, and make-up products, in particular waterproof products, accumulate in the skin folds, and can block the pores of the skin and result in the appearance of spots.

Several types of skin cleansing products, for example, rinsable cleansing anhydrous oils and gels, and foaming creams, lotions and gels, are known.

Rinsable anhydrous oils and gels have a cleansing action by virtue of oils present in these formulations. These oils make it possible to dissolve fatty residues and to disperse make-up pigments. These products are effective and well tolerated. However, they exhibit the disadvantages of being heavy, of not foaming and of not conferring a feeling of freshness on application, which is disadvantageous from a cosmetic viewpoint.

On the other hand, foaming cleansing products have a cleansing action by virtue of the surfactants, which suspend the fatty residues and the pigments of, for example, the make-up products. They are effective and pleasant to use because they foam and because they are easy to remove. Visual as well as sensorial properties of foam are very important. Rapid formation of big volumes of a creamy foam is the important visual requirement, while a dense foam feel, often associated with creaminess, is the most wanted sensorial property.

Recently, cleansing products, in particular facial cleansing compositions, are expected to have not only cleansing effects and foaming ability but also to leave a good feeling on the skin after use, such as a fresh feeling, a not-tight feeling, a skin mildness and moisturizing sensation and a natural feeling.
Most of the foaming face cleansing products which are well-known in the prior art and are actually on the market are based on formulas that contain generally fatty acid salts (soaps) preferably with anionic surfactants of the sulphate type (ie alkylsulfates and alkylether sulfates). However, the drawback to these formulas is that, in order to provide a high level of foaming performance, they need to use high quantities of surfactants (> 20%) and consequently have a high pH (approximatively 9). Those high quantities of soaps and sulphate surfactants may lead to skin irritation and discomfort on the skin after use (tight feeling, dry sensation) - especially with long term use.
Thus, cleansing products which can contain other anionic surfactants than soaps and sulfate surfactants and have both good foamability performance (fast foam start, consistent foam, density, volume, and elasticity) and good feeling of the skin after use are still desired.

### DISCLOSURE OF INVENTION

An objective of the present invention is to provide a composition, in particular a cleansing composition, which can contain anionic surfactants other than soaps and sulfate surfactants and which have good foamability performance and good feeling of the skin after use, such as fresh feeling, a not-tight feeling, skin mildness and moisturizing sensation and natural feeling; the said composition being free of the abovementioned drawbacks.
The inventors have demonstrated that this objective could be achieved with a composition, in particular a cleansing composition, containing, in an aqueous medium, at least:
a) a sulfonate mixture which will be defined in greater detail hereinbelow and
b) at least one polymeric suspension agent.
One subject of the present invention is a composition containing, in an aqueous medium, at least:
1) a sulfonate mixture containing:
   a) an internal olefin sulfonate (A) having 16 carbon atoms and
   b) an internal olefin sulfonate (B) having 18 carbon atoms,
   wherein the weight ratio (A/B) of the component (A) to the component (B) present in the sulfonate mixture is from 75/25 to 90/10, and
   wherein the weight ratio of the hydroxy form compounds present in the internal olefin sulfonates (A) and (B) to the olefin form compounds present in the internal olefin sulfonates (A) and (B) is from 75/25 to 100/0; and
2) at least one polymeric suspension agent selected from starches, carrageenan, xanthan gum, guar gum, and celluloses such as hydroxyethylcellulose and hydroxypropylcellulose; the non-ionic associative polyether-urethanes;
the crosslinked homopolymers and crosslinked copolymers of acrylic acid and/or methacrylic acid and/or their esters, and mixtures thereof. Another subject of the present invention is a composition containing, in an aqueous medium, at least:
1) a sulfonate mixture containing:
   a) an internal olefin sulfonate (A1) having 16 carbon atoms which will be defined in areater detail hereinbelow and
   b) an internal olefin sulfonate (B1) having 18 carbon atoms, which will be defined in greater detail hereinbelow and
      - optionally one or more compounds of formula (A2) and/or (A3) which will be defined in greater detail hereinbelow;
      - optionally one or more compounds of formula (B2) and/or (B3) which will be defined in greater detail hereinbelow;
      wherein:
      - the weight ratio of all the compounds (A1), (A2) and (A3) to all the compounds (B1), (B2) and (B3) (which means (A1+A2+A3)/(B1+B2+B3)) is from 75/25 to 90/10; and
      - the weight ratio of all the compounds (A1) and (B1) to all the compounds (A2), (B2), (A3) and (B3) (which means (A1+B1)/(A2+A3+B2+B3)) is from 75/25 to 100/0;
         and
2) at least one polymeric suspension agent selected from starches, carrageenan, xanthan gum, guar gum, and celluloses such as hydroxyethylcellulose and hydroxypropylcellulose; the non-ionic associative polyether-urethanes; the crosslinked homopolymers and crosslinked copolymers of acrylic acid and/or methacrylic acid and/or their esters, and mixtures thereof.
Preferably, the said compositions contain a physiologically acceptable aqueous medium, and more particularly a cosmetically acceptable aqueous medium Contrary to all expectations, the inventors have found that the use of this cosmetic composition makes it possible to have both good foamability performance (fast foam start, consistent foam density, volume, and elasticity) without using necessarily high amounts of anionic surfactants and a basic pH and good feeling of the skin after use: fresh feeling, a not-tight feeling, skin mildness and moisturizing sensation and natural feeling.
According to another of its aspects, a subject of the invention is also a process for cleansing keratin materials, which consists in applying to the said keratin materials a composition according to the invention, in working the said composition into a foam and then rinsing off the said composition, especially with water.
A subject of the present invention is also the cosmetic use of the composition as defined above, for removing makeup and/or cleansing the skin, the hair and/or mucous membranes, or for skincare.

### BEST MODE FOR CARRYING OUT THE INVENTION

For the purposes of the present invention, the term "physiologically acceptable medium" means a medium that is suitable for the topical administration of a composition. A physiologically acceptable medium is preferably a cosmetically or dermatologically acceptable medium, that is to say a medium which is devoid of unpleasant odour or appearance and which is entirely compatible with the topical administration route. Such a medium is in particular considered as physiologically acceptable when it does not cause the user any unacceptable stinging, tautness or redness.
For the purposes of the present invention, the term "polymeric suspension agent" means any polymeric compound which gels or thickens the composition and also allow to avoid sedimentation of the eventual particles which may be present in the composition according to the present invention.

For the purposes of the present invention, the term "polymeric compound" means any large molecule, or macromolecule, composed of repeated subunits, known as monomers (at least two monomers) which are linked with each other by covalent bonds by chemical reaction.

The compositions of the invention are preferably rinse-off compositions (rinsing with water or with a tonic) and they may be used in the field of the makeup removal and cleansing of facial or bodily skin, the hair, including the scalp, and mucous membranes such as the lips. They may also constitute care products, for instance rinse-off masks (in the usual manner in which these products are used).

### SULFONATE COMPOSITION

The cosmetic composition according to the invention comprises a sulfonate mixture containing:
a) an internal olefin sulfonate (A) having 16 carbon atoms and
b) an internal olefin sulfonate (B) having 18 carbon atoms,
wherein the weight ratio (A/B) of the component (A) to the component (B) present in the sulfonate mixture is from 75/25 to 90/10, and
wherein the weight ratio of the hydroxy form compounds present in the internal olefin sulfonates (A) and (B) to the olefin form compounds present in the internal olefin sulfonates (A) and (B) is from 75/25 to 100/0.

By "internal olefin sulfonate", we mean the reaction product obtained by sulfonation reaction of an internal olefin, followed by a neutralization step and then a hydrolysis step.

By "internal olefin", we mean an olefin in which the double bond C=C is located internally in the chain (which means not located at the extremity of the said chain).

By "hydroxy form compound", we mean the saturated compounds comprising a hydroxy radical and a sulfonate radical, present in the said reaction product.

By "olefin form compound", we mean the unsaturated compounds comprising a C=C double bond and a sulfonate radical, present in the said reaction product.

Preferably, the sulfonate mixture comprises the internal olefin sulfonate (A) and the internal olefin sulfonate (B) in an amount from 50 to 100% by weight relative to the total weight of the sulfonate mixture.

Preferably, the sulfonate mixture of the invention comprises the internal olefin sulfonates (A) and (B) having a sulfonate group on the carbon atom located at C-2 position in a total amount of 28% by weight or less relative to the total weight of the sulfonate mixture. More preferably, it is from 10 to 28% by weight, and better from 15 to 25% by weight.

Preferably, the weight ratio (A/B) of the internal olefin sulfonate (A) to the internal olefin sulfonate (B) present in the sulfonate mixture is from 80/20 to 85/15. Preferably, the weight ratio of the hydroxy form compounds present in the said internal olefin sulfonates (A) and (B) to the olefin form compounds present in the internal olefin sulfonates (A) and (B) is from 80/20 to 95/5.

The internal olefin sulfonate (A) having 16 carbon atoms can therefore contain one or more hydroxyl form compounds and optionally one or more olefin form compounds.

The internal olefin sulfonate (A) having 18 carbon atoms can therefore contain one or more hydroxyl form compounds and optionally one or more olefin form compounds.

The sulfonate mixture used in the context of the present invention can also be defined as comprising:
- one or more compounds of formula (A1): 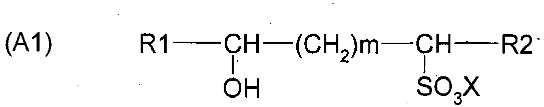 in which:
   - R1 and R2 represent, independently, a hydrogen atom or a branched or linear C1-C14 alkyl radical;
   - m is an integer from 0 to 14,
      R1, R2 and m are such that the said compound(s) (A1) comprise 16 carbon atoms and
   - X represents a cation or a mixture of cations allowing achievement of electroneutrality of the said compound(s) (A1);
- one or more compounds of formula (B1): 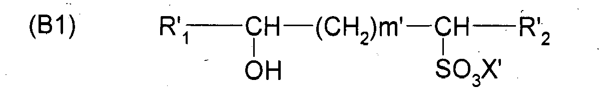 in which:
   - R'1 and R'2 represent, independently, a hydrogen atom or a branched or linear C1-C16 alkyl radical;
   - m' is an integer from 0 to 16,
      R'1, R'2 and m' being such that the said compound(s) (B1) comprise 18 carbon atoms and
   - X' represents a cation or a mixture of cations allowing achievement of electroneutrality of the said compound(s) (B1); and
   - optionally one or more compounds of formula (A2) and/or (A3): 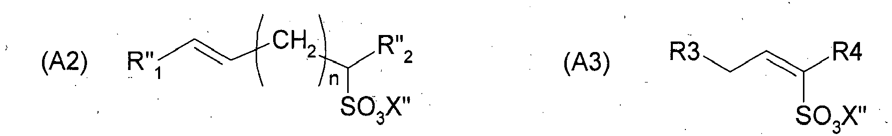 in which:
      - R"1, R"2, R3 and R4 represent, independently, a hydrogen atom or a branched or linear C1-C13 alkyl radical;
      - n is an integer from 0 to 13,
         R"1, R"2, n, R3 and R4 being such that the said compound(s) (A2) and (A3) comprise 16 carbon atoms, and
      - X" represents a cation or a mixture of cations allowing achievement of electroneutrality of the said compound(s) (A2) and/or (A3); and
      - optionally one or more compounds of formula (B2) and/or (B3): 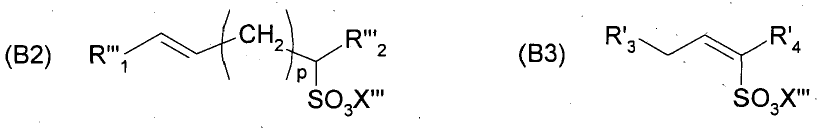 in which:
         - R'''1, R'''2, R'3 and R'4 represent, independently, a hydrogen atom or a branched or linear C1-C15 alkyl radical;
         - p is an integer from 0 to 15,
            R'''1, R'''2, p, R'3 and R'4 being such that the said compound(s) (B2) and (B3) comprise 18 carbon atoms, and
         - X''' represents a cation or a mixture of cations allowing achievement of electroneutrality of the said compound(s) (B2) and/or (B3);
      wherein:
      - the weight ratio of all the compounds (A1), (A2) and (A3) to all the compounds (B1), (B2) and (B3) (which means (A1+A2+A3)/(B1+B2+B3)) is from 75/25 to 90/10; and
      - the weight ratio of all the compounds (A1) and (B1) to all the compounds (A2), (B2), (A3) and (B3) (which means (A1+B1)/(A2+A3+B2+B3)) is from 75/25 to 100/0.

In this definition, the compounds of formula (A1) and (B1) correspond to the hydroxy form compounds.

In this definition, the optional compounds of formula, (A2), (B2), (A3) and (B3) correspond to the olefin form compounds.

In the present application, in the calculation of the weight ratios, by "all the compounds (A1)", we mean all the compounds corresponding to the formula (A1) which are present in the sulfonate mixture. It is the same for the other compounds (A2), (A3), (B1), (B2) and (B3).

Preferably, in the sulfonate mixture according to the invention, the weight ratio of all the compounds (A1), (A2) and (A3) to all the compounds (B1), (B2) and (B3) (which means (A1+A2+A3)/(B1+B2+B3)) is from 80/20 to 85/15.

Preferably, in the sulfonate mixture according to the invention, the weight ratio of all the compounds (A1) and (B1) to all the compounds (A2), (B2), (A3) and (B3) (which means (A1+B1)/(A2+A3+B2+B3)) is from 80/20 to 95/5, preferably from 85/15 to 90/10.

Preferably, the alkyl radicals (ie: R1, R2, R'1, R'2, R"1, R"2, R3, R4, R'''1, R'''2, R'3 and R'4) are linear.

Preferably, the cations X, X', X" and X''' are organic or inorganic. They are preferably selected such that the compounds (A1), (A2), (A3), (B1), (B2) and (B3) are physiologically acceptable, in particular cosmetically acceptable.

In the sense of the invention, by "physiologically acceptable compound", we mean a compound suitable for the topical administration of a composition containing it.

Preferably, the cations are selected from those corresponding to alkaline metals or alkalino-earth metals; in particular, selected from those corresponding to alkaline metals, and better corresponding to sodium (Na⁺).

Preferably, the sulfonate mixture according to the invention comprises at least compounds of formula (A1) in which m=0, the group (CH₂)ₘ being therefore replaced by a carbon-carbon simple bond (C-C).

Preferably, the sulfonate mixture according to the invention comprises at least compounds of formula (B1) in which m'=0, the group (CH₂)_{m'} being therefore replaced by a carbon-carbon simple bond (C-C).

Preferably, when the sulfonate mixture according to the invention comprises also compounds of formula (A2), at least a part of the said compounds is such that n=0, the group (CH₂)ₙ being therefore replaced by a carbon-carbon simple bond (C-C).

Preferably, when the sulfonate mixture according to the invention comprises also compounds of formula (B2), at least a part of the said compounds is such that p=0, the group (CH₂)ₚ being therefore replaced by a carbon-carbon simple bond (C-C).

Preferably, the sulfonate mixture according to the invention, comprises at least compounds of formula (A1) in which R2=CH₃ (the sulfonate group being therefore located on the carbon atom at C2 position) and/or the sulfonate mixture according to the invention, comprises at least compounds of formula (B1) in which R'2=CH₃ (the sulfonate group being therefore located on the carbon atom at C2 position).

Preferably, the sulfonate mixture according to the invention, comprises the compounds having a sulfonate group located on the carbon atom at C2 position (in particular (A1), (B1) and optionally (A2), (B2), (A3), (B3)) in a total amount of 28% by weight or less relative to the total weight of the sulfonate mixture. More preferably, it is from 10 to 28% by weight, and better from 15 to 25% by weight.

Preferably, the sulfonate mixture comprises:
- the compound(s) (A1) in an amount of from 50 to 90% by weight, preferably from 55 to 85% by weight, more preferably from 60 to 80% by weight, relative to the total weight of the sulfonate mixture and/or
- the compound(s) (B1) in an amount of from 5 to 25% by weight, preferably from 10 to 23% by weight, more preferably from 15 to 20% by weight, relative to the total weight of the sulfonate mixture and/or
- the compound(s) (A2) and (A3) in a total amount of from 0 to 20% by weight, preferably from 1 to 20% by weight, more preferably from 5 to 15% by weight, relative to the total weight of the sulfonate mixture and/or
- the compound(s) (B2) and (B3) in a total amount of from 0 to 7% by weight, preferably from 0.5 to 5% by weight, more preferably from 1 to 4% by weight, relative to the total weight of the sulfonate mixture.
The sulfonate mixture according to the invention may be obtained by sulfonation of an internal olefin, followed by a neutralization step then by a hydrolysis step.
A general method of preparation of the internal olefin sulfonate is notably described in the publication 'Hydroxy alkane sulfonate, a new surfactant based on olefins', by Stapersma et al., published in JAOCS, vol. 69, no1 (January 1992).
Advantageously, a composition of the invention comprise preferably from 0.5% to 20% by weight of the sulfonate mixture, more preferably from 1% to 15% by weight, more particularily from 1% to 10% by weight relative to the total weight of the composition.

### POLYMERIC SUSPENSION AGENT

The polymeric suspension agent is selected from starches, carrageenan, xanthan gum, guar gum, and celluloses such as hydroxyethylcellulose and hydroxypropylcellulose; the non-ionic associative polyether-urethanes; the crosslinked homopolymers and crosslinked copolymers of acrylic acid and/or methacrylic acid and/or their esters, and mixtures thereof.

The polymeric suspension agents may be of natural origin. They may then be chosen from water-soluble polysaccharides polymers.
The term "polysaccharide" means any polymer consisting of several saccharides (or monosaccharides) having the general formula:

-[Cₓ(H₂O)_{y})]ₙ- (in which y is generally x - 1)

and linked together via O-oside bonds.
The term "water-soluble polysaccharide " means any polysaccharide which, when introduced into water or into a mixture of water and linear or branched C₂-C₅ monoalcohols, for instance ethanol, isopropanol or n-propanol, without pH modification at 25°C, to a mass concentration equal to 1%, allows the production of a macroscopically homogeneous and transparent solution, i.e. a solution with a minimum light transmission value, at a wavelength equal to 500 nm, through a sample 1 cm thick, of at least 80% and preferably at least 90%.
The water-soluble polysaccharides that may be used in the present invention are especially chosen from starches, carrageenan, xanthan gum, guar gum, and celluloses such as hydroxyethylcellulose and hydroxypropylcellulose, and mixtures thereof.
The polymeric suspension agents according to the invention may be of synthetic origin.
They may be chosen from
- the associative or non-associative crosslinked homopolymers and crosslinked copolymers of acrylic acid and/or methacrylic acid and/or their esters;
- the non-ionic associative polyether-urethanes.
For the purposes of the present invention, the term "associative polymers" means hydrophilic polymers that are capable, in an aqueous medium, of reversibly associating with each other or with other molecules. Their chemical structure more particularly comprises at least one hydrophilic region and at least one hydrophobic region.

The term "hydrophobic group" is understood to mean a radical or polymer comprising a saturated or unsaturated and linear or branched hydrocarbon-based chain, When the hydrophobic group denotes a hydrocarbon-based radical, it comprises at least 10 carbon atoms, preferably from 10 to 30 carbon atoms, in particular from 12 to 30 carbon atoms and more preferentially from 18 to 30 carbon atoms. Preferentially, the hydrocarbon-based group is derived from a monofunctional compound.

By way of example, the hydrophobic group may be derived from a fatty alcohol, such as stearyl alcohol, dodecyl alcohol or decyl alcohol, or else from a polyoxyalkylenated fatty alcohol, such as Steareth-100. It may also denote a hydrocarbon-based polymer, for instance polybutadiene.

For the purposes of the present invention, the term "non associative polymers" means that the polymer does not have the behaviour of an associative polymer as above explained. It is generally a hydrophilic polymer of acrylic acid and/or methacrylic acid and of an ester thereof comprising less than 6 carbon atoms: ie a C₁-C₄ alkylacrylate for example, chosen from methyl acrylate, ethyl acrylate and butyl acrylate.

The copolymers disclosed herein are partially or totally crosslinked with at least one standard crosslinking agent. The at least one crosslinking agent can be chosen, for example, from polyunsaturated compounds, such as polyethylenically unsaturated compounds. For example, these compounds can be chosen from polyalkenyl ethers of sucrose, polyalkenyl ethers of polyols, diallyl phthalates, divinylbenzene, allyl (meth)acrylate, ethylene glycol di(meth)acrylate, methylenebisacrylamide trimethylolpropane tri(meth)acrylate, diallyl itaconate, diallyl fumarate, diallyl maleate, zinc (meth)acrylate, castor oil derivatives and polyol derivatives manufactured from unsaturated carboxylic acids.

As examples of such polymers may be mentioned:
(1) The homopolymers of acrylic acid crosslinked with an allyl ether of pentaerythritol, an allyl ether of sucrose, or an allyl ether of propylene like the Carbomer sold under the commercial name CARBOPOL® by the company LUBRIZOL.
(2) The non-associative crosslinked copolymers of acrylic acid and/or methacrylic acid and esters thereof.

As an example of such non-associative crosslinked copolymers of acrylic acid and/or methacrylic acid and esters thereof, can be mentioned:
i) The crosslinked copolymers of acrylic acid and/or methacrylic acid and of an ester thereof comprising less than 6 carbon atoms and more particulary in the form of an aqueous 30% dispersion as the copolymer sold under the commercial name ACULYN 33® by the company THE DOW CHEMICAL COMPANY and having the INCI name: ACRYLATES COPOLYMER.
ii) The crosslinked copolymers comprising at least one methacrylic acid unit and at least one C₁-C₄ alkyl acrylate unit. These copolymers are described, for example, in Patent Application no WO 01/76552.

As used herein, the crosslinked copolymer comprising at least one methacrylic acid unit and at least one C₁-C₄alkyl acrylate unit means a crossklinked copolymer comprising at least one methacrylic acid unit and at least one alkyl acrylate unit, wherein the alkyl acrylate unit is chosen from C₁-C₄ alkyl acrylates.

In the crosslinked copolymers disclosed herein, the methacrylic acid unit can be present, for example, in an amount ranging from 20% to 80% by weight, such as from 25% to 70% by weight, and further such as from 35% to 60% by weight, relative to the total weight of the copolymer.

In the crosslinked copolymer disclosed herein, the alkyl acrylate unit can be present, for example, in an amount ranging from 15% to 80% by weight, such as from 25% to 75% by weight and further such as from 40% to 65% by weight, relative to the total weight of the copolymer. The alkyl acrylate unit can be chosen, for example, from methyl acrylate, ethyl acrylate and butyl acrylate. In one embodiment, the alkyl acrylate unit is ethyl acrylate.

According to one embodiment, the at least one crosslinked copolymer disclosed herein may be in the form of a dispersion in water. The number-average size of the copolymer particles in the dispersion generally ranges from 10 to 500 nm, for example, from 20 to 200 nm and further, for example, from 50 to 150 nm.

Use can be made, for example, of the crosslinked copolymers comprising at least one methacrylic acid unit and at least one ethyl acrylate unit in the form of an aqueous 30% dispersion manufactured and sold under the name CARBOPOL AQUA SF-1 ® by the company NOVEON.

Amongst the polymeric suspension agents chosen from crosslinked homopolymers and crosslinked copolymers of acrylic acid and/or methacrylic acid and/or their esters can be also mentioned:
(3) The associative anionic crosslinked polymers of acrylic acid and/or methacrylic acid and/or their esters and more particularly those consisting of 95 to 60% by weight of acrylic acid (hydrophilic unit), 4 to 40% by weight of C₁₀-C₃₀ alkyl acrylate (hydrophobic unit), and 0 to 6% by weight of crosslinking polymerizable monomer, or alternatively those consisting of 98 to 96% by weight of acrylic acid (hydrophilic unit), 1 to 4% by weight of C₁₀-C₃₀ alkyl acrylate (hydrophobic unit) and 0.1 to 0.6% by weight of crosslinking polymerizable monomer such as those described above.

Among said above polymers can be cited the products sold by the company GOODRICH under the trade names PEMULEN TR1®, PEMULEN TR2®, CARBOPOL 1382®, and still more preferably PEMULEN TR1®, and the product sold by the company S.E.P.P.I.C. under the name COATEX SX®, and having the INCI name: ACRYLATES/C₁₀-C₃₀ ALKYL ACRYLATE CROSSPOLYMER.

We can also mention the copolymers comprising among their monomers a carboxylic acid with α,β-monoethylenic unsaturation and an ester of a carboxylic acid with α,β-monoethylenic unsaturation and an oxyalkylenated fatty alcohol. Preferably, these compounds also comprise, as monomer, an ester of a carboxylic acid with α,β-monoethylenic unsaturation and a C₁-C₄ alcohol. By way of example of this type of compound, there may be mentioned ACULYN 22® sold by the company THE DOW CHEMICAL COMPANY which is an oxyalkylenated stearyl methacrylate/ethyl acrylate/methacrylic acid terpolymer with the INCI Name ACRYLATES/STEARETH-20 METHACRYLATE COPOLYMER.

We can also mention the crosslinked copolymers of acrylic acid/ vinyl isodecanoate as product sold under the commercial name STABYLEN 30 ® (INCI name: ACRYLATES/VINYL ISODECANOATE CROSSPOLYMER) sold by the company SIGMA 3V.

We can also mention the crosslinked copolymers of vinyl neodecanoate and one or more monomers of acrylic acid, methacrylic acid or one of their simple esters crosslinked with an allyl ether of trimethylolpropane or pentaerythritol as the product sold under the commercial name ACULYN 38 ® (INCI name: ACRYLATES/VINYL NEODECANOATE CROSSPOLYMER) sold by THE DOW CHEMICAL COMPANY.

Another family of polymeric suspension agent which is suitable for the composition of the invention is composed of the non-ionic associative polyether-urethanes.

Non-ionic associative polyether-urethanes are nonionic block copolymers comprising in the chain both hydrophilic blocks usually of polyoxyethylene nature (polyurethanes may also be referred to as polyurethane polyethers), and hydrophobic blocks that may be aliphatic sequences alone and/or cycloaliphatic and/or aromatic sequences.

In particular, these polymers comprise at least two hydrocarbon-based lipophilic chains containing from 6 to 30 carbon atoms, separated by a hydrophilic block, the hydrocarbon-based chains possibly being pendent chains or chains at the end of the hydrophilic block. In particular, it is possible for one or more pendent chains to be provided. In addition, the polymer may comprise a hydrocarbon-based chain at one end or at both ends of a hydrophilic block.

Associative polyurethanes may be block polymers, in triblock or multiblock form. The hydrophobic blocks may thus be at each end of the chain (for example: triblock copolymer containing a hydrophilic central block) or distributed both at the ends and in the chain (for example: multiblock copolymer). These polymers may also be graft polymers or star polymers. Preferably, the associative polyurethanes are triblock copolymers in which the hydrophilic block is a polyoxyethylene chain comprising from 50 to 1000 oxyethylene groups. In general, associative polyurethanes comprise a urethane bond between the hydrophilic blocks, whence arises the name.

As examples of nonionic fatty-chain polyurethane polyethers that may be used in the invention, it is also possible to use Rheolate® FX 1100 (Steareth-100/PEG 136/HDI (hexamethyl diisocyanate) copolymer), Rheolate® 205® containing a urea function, sold by the company Elementis, or Rheolate® 208, 204 or 212.

Mention may also be made of the product Elfacos T210® containing a C12-C14 alkyl chain, and the product Elfacos T212® containing a C16-18 alkyl chain (PPG-14 Palmeth-60 Hexyl Dicarbamate) from Akzo.

The associative polyurethanes that may be used according to the invention are in particular those described in the article by G. Fonnum, J. Bakke and Fk. Hansen - Colloid Polym. Sci., 271, 380-389 (1993).

Even more particularly, according to the invention, use may also be made of a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyether-urethanes are sold especially by the company THE DOW CHEMICAL COMPANY under the names Aculyn 46® and Aculyn 44®. Aculyn 46® is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%); Aculyn 44® is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%)].

Use will be made more particularly of an associative nonionic polyurethane polyether such as the product sold especially by the company Elementis under the name Rheolate FX 1100®, which is a polycondensate of polyethylene glycol containing 136 mol of ethylene oxide, of stearyl alcohol polyoxyethylenated with 100 mol of ethylene oxide and of hexamethylene diisocyanate (HDI) with a weight-average molecular weight of 30,000 (INCI name: PEG-136/Steareth-100I/SMDI Copolymer).

According to a preferred embodiment of the invention, the polymeric suspension agent will be chosen from the non-associative crosslinked copolymers of acrylic acid and/or methacrylic acid and esters thereof and more preferably:
- the crosslinked copolymers comprising at least one methacrylic acid unit and at least one ethyl acrylate unit and more particularily in the form of an aqueous 30% dispersion manufactured and sold under the name CARBOPOL AQUA SF-1 ® by the company NOVEON;
- the crosslinked copolymers of acrylic acid and/or methacrylic acid and of an ester thereof comprising less than 6 carbon atoms and more particularily in the form of an aqueous 28% dispersion as the copolymer sold under the commercial name ACULYN 33® by the company THE DOW CHEMICAL COMPANY and having the INCI name: ACRYLATES COPOLYMER and their mixtures.

The polymeric suspension agent may be included in the composition according to the present invention preferably from 0.1% to 10% by weight relative to the total weight of the composition, more preferably from 1 to 8 % by weight, and more particularily from 2 to 5 % by weight relative to the total weight of the composition.

According to one embodiment of the invention, the compositions will be soap-free and/or sulfate surfactant-free.

For the purposes of the present invention, the term "soap-free" means that the composition contains less than 1.0% by weight relative to the total weight of the composition of fatty acid salt (soap) and preferably less than 0.5% by weight and more preferably less than 0.1% by weight.

For the purposes of the present invention, the term "sulfate surfactant-free" means that the composition contains less than 1.0% by weight relative to the total weight of the composition of surfactant of the type sulphate (ie alkylsulfates, alkylether sulfates) and preferably less than 0.5% by weight and more preferably less than 0.1% by weight.

According to one embodiment of the invention, the compositions will be both soap-free and sulfate surfactant-free.

According to one embodiment of the invention, the compositions may further contain other surfactants which are selected from anionic surfactants, non-ionic surfactants, amphoteric or zwitterionic surfactants, cationic surfactants and their mixtures.

### NON-IONIC SURFACTANTS

According to one embodiment of the invention, the compositions may further contain at least one non-ionic surfactant.

The nonionic surfactants that may be present in the composition of the invention may be chosen especially from oxyalkylenated glycerol esters, oxyalkylenated sugar esters, alkyl polyglucosides (APG) and mixtures thereof. They are preferably alkyl polyglucosides.

The oxyalkylenated glycerol esters are in particular the polyoxyethylenated derivatives of esters of glycerol and of a fatty acid and of their hydrogenated derivatives. These oxyalkylenated glycerol esters can be chosen, for example, from esters of glycerol and of fatty acids which are hydrogenated and oxyethylenated, such as PEG-200 hydrogenated glyceryl palmate, sold under the name Rewoderm LI-S 80 by Goldschmidt; oxyethylenated glycerol cocoates, such as PEG-7 glyceryl cocoate, sold under the name Tegosoft GC by Goldschmidt, and PEG-30 glyceryl cocoate, sold under the name Rewoderm LI-63 by Goldschmidt; and mixtures thereof.

The oxyalkylenated sugar esters are in particular polyethylene glycol ethers of fatty acid and sugar esters. These oxyalkylenated sugar esters can be chosen, for example, from oxyethylenated glucose esters, such as PEG-120 methyl glucose dioleate, sold under the name Glucamate DOE 120 by Amerchol.

According to a preferred embodiment of the invention, the nonionic surfactant is an alkyl polyglucoside which may be chosen especially from decylglucoside, caprylyl/capryl glucoside, laurylglucoside, cocoylglucoside and caprylylglucoside, and mixtures thereof.

The alkyl poly glucoside are preferably selected from those corresponding to the formula (I):

R-O-Gx' (I)

wherein R is a C₆-C₄₀, preferably C₈-C₃₄ alkyl group, G is a moiety derived from a reducing saccharide containing from 5 to 6 carbon atoms, preferably a glucose unit, and x' represents the average degree of polymerisation of the alkyl poly glucoside.

For a particular alkyl polysaccharide molecule, x' can only assume integral values. In any physical sample of alkyl poly glucosides, there will generally be molecules having different values of x'. The physical sample can be characterized by the average value of x', which can assume non-integral values. In the specification, the values of x' are to be understood to be average values.

The polysaccharide hydrophilic portion of the alkyl polyglucoside contains preferably from about 1 to about 10, more preferably from 1.4 to 3, saccharide units on average.

The saccharide unit may be galactoside, glucoside, lactoside, fructoside, glucosyl, fructosyl, lactosyl, and/or galactosyl units, and preferably glucoside unit.

Mixtures of these saccharide moieties may be used in the alkylpolysaccharide. Glucoside is the preferred saccharide moiety. Other saccharide moieties will act similarly, but because glucoside is the preferred saccharide moiety, the remaining disclosure will focus on the alkylpolyglucoside.

The hydrophilic group on the alkyl polyglucoside is an alkyl group, either saturated or unsaturated, branched or unbranched, containing from about 6 to about 40 carbon atoms on average. Preferably the alkyl group is primarily a straight chain saturated C₈ to C₃₄ alkyl group.

Useful alkyl polyglucosides of the current invention are also disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3, 4-, etc. positions thus giving a glucose or galatose as opposed to a glucoside or galactoside). The intersaccharide bonds can be, e.g., between the one position of the additional saccharide units and the 2-, 3-, 4-, and/or 6- positions on the preceding saccharide units.

Examples of alkyl polyglucoside compounds include but are not limited to: caprylyl/capryl glucoside, decyl glucoside, lauryl glucoside, octyl glucoside, sodium lauryl glucose carboxylate (and) lauryl glucoside, and coco glucoside.

Typically, the alkyl poly glucoside compounds are selected from the group consisting of coco glucoside, lauryl glucoside, and decyl glucoside, and more typically coco glucoside and decyl glucoside.

The alkylpolyglucosides may be chosen, for example, from
- Decylglucoside (alkyl-C₉/C₁₁-polyglucoside (1.4)), such as the product sold under the name MYDOL 10® by the company Kao Chemicals or the product sold under the name PLANTACARE 2000 UP® by the company Cognis;
- Caprylyl/capryl glucoside, such as the product sold under the name PLANTACARE K 3711® by the company Cognis;
- Laurylglucoside, such as the product sold under the name PLANTACARE 1200 UP® by the company Cognis;
- Cocoglucoside, such as the product sold under the name PLANTACARE 818 UP® by the company Cognis;
- Caprylylglucoside, such as the product sold under the name PLANTACARE 810 UP® by the company Cognis; and mixtures thereof.
- For Octylglucoside: REWOSAN® sold by Rewo,
- For Sodium Lauryl Glucose Carboxylate: PLANTAPON® LGC SORB.

Preferably, mention can be made of Caprylyl/capryl glucoside, such as the product sold under the name PLANTACARE K 3711® by the company Cognis.

Advantageously, a composition of the invention comprises preferably from 0.1% to 20% by weight of the non-ionic surfactant more preferably from 0.5% to 15% by weight, more particularily from 1% to 8% by weight relative to the total weight of the composition.

### AMPHOTERIC OR ZWITTERIONIC SURFACTANTS

According to one embodiment of the invention, the compositions may further contain at least one amphoteric or zwitterionic surfactant.

The amphoteric or zwitterionic surfactants can be chosen, for example, from betaines, N-alkylamidobetaines and derivatives thereof, sultaines, alkyl polyaminocarboxylates, alkylamphoacetates, and mixtures thereof.

Mention may in particular be made, as betaines, of alkyl betaines, such as, for example, Coco betaine, such as the product sold under the name Dehyton AB-30® by the company Cognis, Lauryl betaine, such as the product sold under the name Genagen KB® by the company Clariant, oxyethylenated (10 EO) lauryl betaine, such as the product sold under the name Lauryl Ether (10 EO) Betaine® by the company Shin Nihon Rica, or oxyethylenated (10 EO) stearyl betaine, such as the product sold under the name Stearyl Ether (10 EO) Betaine® by the company Shin Nihon Rica.

Mention may be made, among N-alkylamidobetaines and derivatives thereof, for example, of Cocamidopropyl betaine, sold under the name LEBON 2000 HG® by the company Sanyo or sold under the name EMPIGEN BB® by the company Albright & Wilson, or lauramidopropyl betaine, sold under the name Rewoteric AMB12P® by the company Witco.

Mention may be made, as sultaines, of hydroxysultaines, cocamidopropyl hydroxysultaine, such as the product sold under the name REWOTERIC AM CAS® by the company Goldschmidt-Degussa or the product sold under the name CROSULTAINE C-50® by the company Croda.

Mention may be made, as alkyl polyaminocarboxylates (APACs), of sodium cocoylpolyaminocarboxylate, sold under the names AMPHOLAK 7 CX/C® and Ampholak 7 CX® by the company Akzo Nobel, sodium stearylpolyamidocarboxylate, sold under the name AMPHOLAK 7 TX/C® by the company Akzo Nobel, or sodium carboxymethyloleylpolypropylamine, sold under the name Ampholak XO7/C® by the company Akzo Nobel.

Mention may be made, as alkylamphoacetates, for example, of N-disodium N-cocoyl-N-carboxymethoxyethyl-N-(carboxymethyl)ethylenediamine (CTFA name: disodium cocoamphodiacetate), such as the product sold under the name Miranol C2M Concentré NP® by the company Rhodia, N-sodium N-cocoyl-N-hydroxyethyl-N-(carboxymethyl)ethylenediamine (CTFA name: sodium cocamphoacetate) or sodium cocoamphohydroxypropylsulfonate, sold under the name Miranol CSE by the company Rhodia.

Preferably, the amphoteric or zwitterionic surfactant is chosen from betaines, and especially alkylbetaines and more particularily Coco betaine, such as the product sold under the name DEHYTON AB-30® by the company Cognis.

Advantageously, a composition of the invention comprise preferably from 0.1% to 20% by weight of the amphoteric or zwitterionic surfactant, more preferably from 0.5% to 15% by weight, more particularily from 1% to 10% by weight relative to the total weight of the composition.

### FOAM-ENHANCING AGENTS OR FOAM BOOSTERS

According to one embodiment of the invention, the compositions may further contain at least one foam-enhancing agent or one foam booster.

The foam-enhancing agents or foaming boosters which are suitable according to the invention can be preferably selected from:
- the fatty alcohols;
- the polyalkyleneglycols;
- the polyglyceryl fatty acid esters;
- the polyalkyleneglycol ethers of alkylglucose;
- the celluloses;
- the crosslinked polymers formed by the reaction of an C₁₀-C₁₈ alkylglucoside with 1,3-dichloro-2-propanol, and sulfonated with 3-chloro-2-hydroxypropyl sulfonate;
- the fatty acid alkanolamides;
- and their mixtures.
   A) The fatty alcohols are preferably those comprising a linear saturated alkyl chain having from 10 to 18 carbon atoms as for instance lauryl alcohol, myristyl alcohol, cetyl alcohol, stearyl alcohol and their mixtures like mixtures of cetyl alcohol and stearyl alcohol (cetearyl alcohol);
   B) The polyalkyleneglycols are preferably those of formula (II)

      H-[O-R-]n-OH (II)

      in which
      - R represents a linear alkyl chain containing from 1 to 4 carbon atoms, and
      - n is an integer ranging from 4 to 100000 and advantageously from 4 to 50000.

      According to one particular embodiment of the invention, a polyalkylene glycol in accordance with the invention is a polyethylene glycol.
      According to one preferred embodiment, a polyethylene glycol in accordance with the invention may be chosen from PEG-8 such as the product sold under the trade name Polyethylene Glycol 400 DUB® by the company Clariant, PEG-45M such as the product sold under the trade name POLYOX WSR N 60 K ® by the company Dow Chemical.
   C) The polyglyceryl fatty acid esters are preferably monoesters of one fatty acid comprising a saturated alkyl chain having from 10 to 18 carbon atoms and a polyglycerol having from 2 to 30 moles of glycerol groups.
      According to one preferred embodiment, a polyglycerolated ester in accordance with the invention may be chosen from POLYGLYCERYL-2 LAURATE such as the product sold under the trade name SUNSOFT Q-12D-C® by the company TAIYO KAGAKU.
   D) The polyalkyleneglycol ethers of alkylglucose and preferably polyC₁-C₄alkyleneglycol ethers of C₁-C₄alkylglucose especially Methyl Gluceth-10 as the product sold under the trade name GLUCAM E-10 HUMECTANT® by the company LUBRIZOL and Methyl Gluceth-20 as the product sold under the trade name GLUCAM E-20 HUMECTANT® by the company LUBRIZOL.
   E) The celluloses are preferably polyalkyleneglycol ethers of alkylcellulose like hydroxypropyl methylcellulose as the product sold under the trade name BENECEL K100M HYDROXYPROPYLMETHYL CELLULOSE® by the company ASHLAND.
   F) the crosslinked polymers formed by the reaction of an C₁₀-C₁₈ alkylglucoside with 1,3-dichloro-2-propanol, and sulfonated with 3-chloro-2-hydroxypropyl sulfonate are selected preferably from the polymers having their respective INCI name:
      - SODIUM HYDROXYPROPYLSULFONATE LAURYLGLUCOSIDE CROSSPOLYMER sold under the trade name POLYSUGA NATE 160P® by the company COLONIAL CHEMICAL INC.
      - SODIUM HYDROXYPROPYLSULFONATE COCOGLUCOSIDE CROSSPOLYMER sold under the trade name POLY SUGANATE 124P® by the company COLONIAL CHEMICAL INC.
      - SODIUM HYDROXYPROPYLSULFONATE DECYLGLUCOSIDE CROSSPOLYMER sold under the trade name POLY SUGANATE 100P® by the company COLONIAL CHEMICAL INC.
   G) the fatty acid alkanolamides are preferably selected from C₁₂-C₁₈ fatty acid alkanolamides like Cocamide MEA as the product sold under the trade name COMPERLAN CMEA® by the company BASF.

The most preferred foam boosters will be selected from the polylethyleneglycols and polyglyceryl fatty acid esters and more particularily selected from PEG-45M and POLYGLYCERYL-2 LAURATE.

Advantageously, a composition of the invention comprise preferably from 0.05% to 10% by weight of the foam-enhancing agent or foam booster, more preferably from 0.1% to 5% and much more preferably from 0.2% to 3% by weight by weight relative to the total weight of the composition. To be validated

According to one particular embodiment of the invention, the composition contains in an aqueous medium:
a) at least one sulfonate mixture as above defined; and
b) at least one polymeric suspension as above defined; and
c) at least one non-ionic surfactant and preferably one alkylpolyglucoside surfactant as above defined; and
d) at least one amphoteric or zwitterionic surfactant as above defined; and
e) at least one enhancing-foam agent or foam booster as above defined.

According to one particular embodiment of the invention, the composition contains in an aqueous medium:
a) at least one sulfonate mixture as above defined in an amount of from 0.5% to 20% by weight relative to the total weight; and
b) at least one polymeric suspension as above defined in an amount from 0.1% to 10% by weight relative to the total weight of the composition; and
c) at least one non-ionic surfactant and preferably one alkylpolyglucoside surfactant as above defined in an amount from 0.1% to 20% by weight relative to the total weight of the composition; and
d) at least one amphoteric or zwitterionic surfactant as above defined in an amount of from 0.1% to 20% by weight relative to the total weight of the composition; and
e) at least one enhancing-foam agent or foam booster as above defined in an amount of from 0.05% to 10% by weight relative to the total weight of the composition.

According to one particular embodiment of the invention, the composition contains in an aqueous medium:
a) at least one sulfonate mixture as above defined in an amount of from 1% to 15% by weight relative to the total weight; and
b) at least one polymeric suspension as above defined in an amount from 1% to 8% by weight relative to the total weight of the composition; and
c) at least one non-ionic surfactant and preferably one alkylpolyglucoside surfactant as above defined in an amount from 0.5% to 15% by weight relative to the total weight of the composition; and
d) at least one amphoteric or zwitterionic surfactant as above defined in an amount of from 0.15% to 15% by weight relative to the total weight of the composition; and
e) at least one enhancing-foam agent or foam booster as above defined in an amount of from 0.1 % to 5% by weight relative to the total weight of the composition.

According to one particular embodiment of the invention, the composition contains in an aqueous medium:
a) at least one sulfonate mixture as above defined in an amount of from 1% to 10% by weight relative to the total weight; and
b) at least one polymeric suspension as above defined in an amount from 2% to 5% by weight relative to the total weight of the composition; and
c) at least one non-ionic surfactant and preferably one alkylpolyglucoside surfactant as above defined in an amount from 1% to 8% by weight relative to the total weight of the composition; and
d) at least one amphoteric or zwitterionic surfactant as above defined in an amount of from 1% to 10% by weight relative to the total weight of the composition; and
e) at least one enhancing-foam agent or foam booster as above defined in an amount from 0.2% to 3% by weight relative to the total weight of the composition.

According to one preferred embodiment of the invention, those specific compositions are soap-free and/or sulfate surfactant-free and preferably both soap-free and sulfate surfactant-free.

### AQUEOUS PHASE

The composition according to the invention comprises an aqueous medium or aqueous phase, i.e. a medium comprising an amount of water of at least 50% by weight, preferably ranging from 50% to 95% by weight and better still from 60% to 90% by weight relative to the total weight of the composition.

The aqueous phase of the compositions according to the invention may contain, besides water, one or more solvents chosen from monoalcohols comprising from 1 to 6 carbon atoms, and polyols, and mixtures thereof. A monoalcohol that may especially be mentioned is ethanol. Examples of polyols that may especially be mentioned include glycerol; glycols such as butylene glycol, isoprene glycol or propylene glycol, polyethylene glycols such as PEG-8; sorbitol; dialkyleneglycols as dipropyleneglycol; sugars such as glucose, fructose, maltose, lactose and sucrose; and mixtures thereof.

When they are present, the amount of monoalcohols and of polyols in the composition of the invention may range, for example, from 0.01% to 30% by weight, preferably from 2% to 25% by weight and better still from 4% to 20% by weight relative to the total weight of the composition.

The pH of the composition according to the invention is preferably from 3.0 to 7.0 and more preferably from 3.0 to 6.5.

### ADDITIVES

The composition according to the invention may contain various additives, chosen from those conventionally used in skincare or makeup-removing products, insofar as these additives and the amounts thereof do not harm the desired qualities for the composition according to the invention.

The cleansing composition in accordance with the present invention may thus comprise the following additives: co-surfactants; oil; preserving agents; sequestrants (EDTA and salts thereof); alkalinizing or acidifying agents antioxidants; fragrances; dyestuffs; encapsulated or non-encapsulated pigments or soluble dyes; anionic, nonionic, cationic or amphoteric, polymers, exfoliating agents, peeling agents as citric acid, glycolic acid, hyaluronic acid; fillers, propellants.

The amounts of these various adjuvants are those conventionally used in the field under consideration, for example from 0.01% to 20% of active material of the total weight of the composition. These adjuvants and the amounts thereof should be such that they do not modify the property desired for the composition of the invention.

The composition may also comprise a polymeric quaternary ammonium salts . These compounds are conditioning agents, i.e. they produce a comfortable sensation of softness on the skin (moisturization maintenance).

The polymeric quaternary ammonium salts are cationic or amphoteric polymers containing at least one quaternized nitrogen atom. Polymeric quaternary ammonium salts that may especially be mentioned include the Polyquaternium products (CTFA name), which afford softness and creaminess to the foaming cream. These polymers may preferably be chosen from the following polymers:
Polyquaternium 5, such as the product Merquat 5® sold by the company Nalco;
Polyquaternium 6, such as the product Salcare SC 30® sold by the company Ciba, and the product Merquat 100® sold by the company Nalco;
Polyquaternium 7, such as the products Merquat S®, Merquat 2200 and Merquat 550 sold by the company Nalco, and the product Salcare SC 10® sold by the company Ciba;
Polyquaternium 10, such as the product Polymer JR400® sold by the company Amerchol;
Polyquaternium 11, such as the products Gafquat 755®, Gafquat 755N® and Gafquat 734® sold by the company ISP;
Polyquaternium 15, such as the product Rohagit KF 720 F® sold by the company Röhm;
Polyquaternium 16, such as the products Luviquat FC905®, Luviquat FC370®,
Luviquat HM552® and Luviquat FC550® sold by the company BASF;
Polyquaternium 22, such as the product Merquat 280® sold by the company Nalco;
Polyquaternium 28, such as the product Styleze CC10® sold by the company ISP;
Polyquaternium 39, such as the products Merquat Plus 3330® and Merquat
3330PR® sold by the company Nalco;
Polyquaternium 44, such as the product Luviquat Care® sold by the company BASF;
Polyquaternium 46, such as the product Luviquat Hold® sold by the company BASF;
Polyquaternium 47, such as the product Merquat 2001® sold by the company Nalco.

Preferably, the quaternary ammonium salts are chosen from Polyquaternium-7, Polyquaternium-10, Polyquaternium-39 and Polyquaternium-47, and mixtures thereof.

The polymeric quaternary ammonium salts may be in an (active material) amount ranging, for example, from 0.01% to 5% by weight and better still from 0.05% to 1% by weight relative to the total weight of the composition.

As an example of a particular conditioning agent, mention may be made of Polyquaternium-39, sold especially by the company Nalco under the names Merquat Plus 3330® and Merquat 3330PR®.

As an example of a particular conditioning agent, mention may be made of olyquaternium 10, such as the product Polymer JR400® sold by the company Amerchol.

### GALENIC FORMS

The compositions according to the present invention may be under the form of any galenic form generally used in the products for removing makeup and/or for cleansing the skin, the hair and/or mucous

They may be conditioned in various types of devices as tubes, bottles. They may be conditioned under a pressurized form as aerosols or non-aerosol pumps.

### EXAMPLES

### Example 1 of preparation of a sulfonate mixture

### Example A: Synthesis of C₁₆ internal olefins

7000 g (28.9 moles) of 1-hexadecanol (trade name: KALCOL 6098® of the company Kao Corporation), and, as a solid acid catalyst, 700 g (10% by weight relative to the weight of the raw material alcohol) of alumina (STREM Chemicals, Inc.) were placed into a flask with a stirrer, and reactions were allowed to proceed for three hours at 280°C while stirring and passing nitrogen (7000mL/minute) through the system. The alcohol conversion ratio was 100% and the purity of C₁₆ internal olefin was 99.6% after the completion of the reaction. The resulting crude internal olefin was transferred to a distillation flask and distilled at from 136 to 160°C/4.0 mmHg, whereby 100% of pure internal olefin having 16 carbon atoms was obtained. The double bond distribution in the resulting internal olefin was less than 1% by weight at C-1position, 27% by weight at C-2 position, 23% weight at C-3 position, 18% weight at C-4position, 16% weight at C-5 position, 8% weight at C-6 position, and 7% weight in total at C-7 and C-8 positions.

### Example B: Synthesis of C₁₈ internal olefins

7000 g (25.9 moles) of 1-octadecanol (trade name: KALCOL 8098® of Kao Corporation), and, as a solid acid catalyst, 700 g (10% by mass relative to the raw material alcohol) of alumina (STREM Chemicals, Inc.) were placed into a flask with a stirrer, and reactions were allowed to proceed for 15 hours at 280°C while stirring and passing nitrogen (7000mL/minute) through the system. The alcohol conversion ratio was 100% and the purity of C₁₈ internal olefin was 98.2% after the completion of the reaction. The resulting crude internal olefin was transferred to a distillation flask and distilled at from 148 to 158°C/0.5 mmHg, whereby 100% of pure internal olefin was obtained. The double bond distribution in the resulting internal olefin was less than 1% weight at C-1 position, 19% weight at C-2 position, 18% weight at C-3 position, 17% weight at C-4 position, 15% weight at C-5 position, 12% by mass at C-6 position, 9% weight at C-7 position, and 10% weight in total at C-8 and C-9 positions.

### Example C: Production of the mixture C₁₆ internal olefins and C₁₈ internal olefins

11.9kg of the C₁₆ internal olefin (Example A) and 3.1kg of the C₁₈ internal olefin (Example B) were mixed to produce 15.0kg of C₁₆/C₁₈ internal olefins (mass ratio 79.4/20.6).The double bond distribution of the resulting internal olefin was 1% weight at a C-1position, 25% weight at a C-2 position, 22% weight at a C-3 position, 18% weight at a C-4position, 16% weight at a C-5 position, 9% weight at a C-6 position, 4% weight at a C-7 position, 4% weight at a C-8 position, and 1% weight at a C-9 position.

### Synthesis of the sulfonate mixture

Using a thin film sulfonation reactor having an outer jacket, the sulfonation reaction of the internal olefin having 16 and 18 carbon atoms (the content of an internal olefin in which a double bond was present at a C-2 position is 25% weight) obtained in Example C was carried out by passing through sulfur trioxide gas, while passing cooling water of 10°C through the outer jacket of the reactor.

The molar ratio of SO₃/internal olefin for the sulfonation reaction was set at 1.10. The resulting sulfonation product was added to an alkaline aqueous solution prepared with 1.11 times the molar amount of sodium hydroxide relative to the theoretical acid value, followed by neutralization at below 30°C for less than 0.5 hour while stirring. The resulting neutralized product was hydrolyzed by heating at 180°C for one hour in an autoclave. And then, the hydrolyzed product was decolorized by hydrogen peroxide, whereby a sodium C₁₆/C₁₈ internal olefin sulfonate mixture was obtained.

The weight ratio of hydroxy form compounds (sodium hydroxyalkane sulfonate)/olefin form compounds (sodium olefin sulfonate) in the obtained sodium internal olefin sulfonate mixture was 87/13. Also, the content of the raw material internal olefin contained in the obtained sodium internal olefin sulfonate mixture was 1.3% by weight, while the content of inorganic compounds therein was 4.7% by weight. Also, the content of compounds having a sulfonate group located at a C-2 position, present in the internal olefin sulfonate mixture was 16% by weight.

### Examples 2 to 5

The following compositions were prepared. The amounts of the ingredients in an active material content were expressed in percentages by weight relative to the total weight of the composition.

**Table 1**

| **Phase** | **Ingredients** | **Example 2 (invention)** | **Example 3 (Invention)** | **Example 4 (Invention)** | **Example 5 (outside the Invention)** |
|---|---|---|---|---|---|
| A1 | Internal olefins sulfonates composition of example 1 | 8.3 | 8.3 | 8.3 | - |
| A2 | Water | qs | Qs | qs | qs |
| A3 | Acrylates Copolymer (ACULYN 33®) | 3.0 | - | - | - |
| | Acrylates Copolymer (CARBOPOL AQUA SF1®) | | 3.0 | 3.0 | 3.0 |
| B1 | Caprylyl/Capryl Glucoside (PLANTACARE K 3711®) | 4.0 | 4.0 | - | - |
| B2 | Water | qs | Qs | qs | qs |
| B3 | Potassium Hydroxide | 0.81 | 0.81 | - | - |
| B4 | Cocobetaine | 3.4 | 3.4 | - | - |
| C | Dipropylene Glycol | 2.0 | 2.0 | 2.0 | 2.0 |
| | Polyglyceryl-2 Laurate (SUNSOFT Q-12D-C®) | 0.5 | 0.5 | - | - |
| | Glycerin | 10.0 | 10.0 | 10.0 | 10.0 |
| | PEG-45M (POLYOX WSR N 60 K®) | 0.1 | 0.1 | - | - |
| | Phenoxyethanol | 0.5 | 0.5 | 0.5 | 0.5 |
| | pH | 6.5 | 6.5 | 6.5 | 6.5 |
| Foam quality | | ++++ | +++ | +++ | ++ |
| Skin feel after rinsing off | | good film feel, not tight | fresh feel, not tight | fresh feel, not tight | not tight |

### Preparation of the compositions

The phase A comprising the phases A1, A2 and A3 was prepared by mixing with a propeller the phase A1 and A2 at 70-75°C and the phase A3 was introduced little by little into the mixture.

The phase B comprising the phases B1, B2, B3 and B4 was prepared by mixing with a stir bar the phase B1 and B2 then B3 was added to the mixture which was neutralized. The phase B4 was introduced into the mixture with a stir bar.

The phase A was mixed to the phase B with propeller at 70-75°C.

The ingredients of the phase C were mixed with a stir bar at 70-75°C. The phase C was introduced little by little into the mixture A + B with propeller at 70-75°C. The resulting composition was cooled down the room temperature.

The evaluation of both foam quality and after rinsing off skin feel was performed by adding 1 g of product to the hand and systematically creating a foam by introducing tap water. The prototype was mixed for 2 minutes by periodically adding water. After rinsing, the after skin rinse off feel was also evaluated with a particular focus on whether the performance was squeaky, dry/tight or moisturized feeling. Biore skincare facial wash moisture was used as the positive control.
++++ = very good foam quality regarding bubble size, volume and density.
+++ = good foam quality regarding bubble size, volume and density.
++ = moderate foam quality regarding bubble size, volume and density.

We observed that the compositions 2, 3 and 4 according to the invention containing the sulfonate composition have a better foam quality compared to the composition 5 which does not contain the sulfonate composition.

### Example 6: Cleansing composition with peeling effect

**Table 2**

| **Phase** | **Ingredients** | **Example 6 (invention)** |
|---|---|---|
| A1 | Internal olefin sulfonates composition of example 1 | 8.3 |
| A2 | Water | Qs |
| A3 | Acrylates Copolymer (ACULYN 33®) | 3.0 |
| B1 | Caprylyl/Capryl Glucoside (Plantacare K 3711®) | 4.0 |
| B2 | Water | Qs |
| B3 | Potassium Hydroxide | 0.81 |
| B4 | Cocobetaine | 3.4 |
| C | Dipropylene glycol (DIPROPYLENE GLYCOL CARE® of BASF) | 2.0 |
| | Polyglyceryl-2 Laurate (SUNSOFT Q-12D-C®) | 0.5 |
| | Glycerin | 10.0 |
| | Citric Acid | 2.1 |
| | Glycolic Acid | 5.0 |
| | PEG-45M (POLYOX WSR N 60 K ®) | 0.1 |
| | Phenoxyethanol | 0.5 |
| | pH | 3.5 |
| Foam quality | | +++ |
| Skin feel after rinsing off | | fresh feel, not tight |

The composition 6 was prepared according to the same method of preparation of the examples 2 to 5.

### Comparative tests of soap-free formulations: examples 2 and 3 (invention) and example 7 (outside the invention)

A simple foaming test was conducted to examine the soap-free and sulphate-free example 2 and 3 according to the invention compared to the soap-free example 7 (outside the invention) which contains a sulfate surfactant.

The foaming process was conducted as follows: 1 g of foaming cleanser was well dissolved in 20 g of tap water. Foam was generated systematically using a cappuccino (HARIO) machine for 20 seconds by placing the tip in the center of the beaker. The foam density (g/L) was measured with a specific container, and weighed just after the foam was generated.

The method was repeated three times for each cleanser, and the data is tabulated in the Table below. A high foam density correlates to a smaller bubble size, which can possibly be linked to better foaming performance. The composition 7 was prepared according to the same method of preparation of the examples 2 to 5.

**Table 3**

| **Ingredients** | **Example 2 (invention)** | **Example 3 (invention)** | **Example 7 (outside the invention)** |
|---|---|---|---|
| Internal olefins sulfonates composition of example 1 | 8.3 | 8.3 | - |
| Sodium Lauryl ether sulphate (2 OE) | - | - | 8.3 |
| Water | qs 100 | qs 100 | qs 100 |
| Octane-1,2-diol | - | 0.3 | 0.3 |
| Acrylates Copolymer (ACULYN 33®) | 3.0 | - | |
| Acrylates Copolymer (CARBOPOL AQUA SF1®) | - | - | 3.0 |
| Caprylyl/Capryl Glucoside (PLANTACARE K 3711®) | 4.0 | - | - |
| Potassium Hydroxide | 0.81 | - | - |
| Cocobetaine | 3.4 | - | 3.4 |
| Dipropyleneglycol | 2.0 | 2.0 | - |
| Polyglyceryl-2 Laurate (SUNSOFT Q-12D-C®) | 0.5 | - | - |
| Glycerin | 10.0 | 10.0 | - |
| PEG-8 (PEG-400 ®) | - | - | 5.0 |
| PEG-120 METHYL GLUCOSE DIOLEATE (GLUCAMATE DOE-120 THICKENER®) | - | - | 0.75 |
| PEG-45M (POLYOX WSR N 60 K ®) | 0.1 | - | - |
| NaCl | - | - | 0,3 |
| Hexyleneglycol | - | - | 1.0 |
| Phenoxyethanol | 0.5 | 0.5 | 0.5 |
| Sodium Benzoate | - | - | 0.3 |
| pH | 6.5 | 6.5 | 5.8 |
| **Foam density (g/L)** | **136,07 ±0.34** | **124,07 ±2,14** | **92,00 ± 3,27** |
| **Foam quality (made by hand)** | **++++** | **+++** | **++** |
| **Skin feel after rinsing off** | **good film feel, not tight** | **moisture feeling** | **fresh feeling** |

We observed that compositions 2 and 3 according to the invention containing the sulfonate composition and the polymeric suspending agent have a better foam density compared to the composition 7 containing a sulfate surfactant instead of the sulfonate mixture.

## Claims

1. Composition containing, in an aqueous medium, at least:
1) a sulfonate mixture containing:
a) an internal olefin sulfonate (A) having 16 carbon atoms and
b) an internal olefin sulfonate (B) having 18 carbon atoms,
- wherein the weight ratio (A/B) of the component (A) to the component (B) present in the sulfonate mixture is from 75/25 to 90/10, preferably from 80/20 to 85/15, and
- wherein the weight ratio of the hydroxy form compounds present in the internal olefin sulfonates (A) and (B) to the olefin form compounds present in the internal olefin sulfonates (A) and (B) is from 75/25 to 100/0, preferably from 80/20 to 95/5; and
2) at least one polymeric suspension agent selected from:
- starches, carrageenan, xanthan gum, guar gum, and celluloses such as hydroxyethylcellulose and hydroxypropylcellulose, and mixtures thereof;
- the non-ionic associative polyether-urethanes;
- the crosslinked homopolymers and crosslinked copolymers of acrylic acid and/or methacrylic acid and/or their esters.

2. Composition according to claim 1, wherein the sulfonate mixture comprises the internal olefin sulfonates (A) and (B) having a sulfonate group on the carbon atom located at C-2 position in a total amount of 28% by weight or less relative to the total weight of the sulfonate mixture, and preferably, from 10 to 28% by weight, and better from 15 to 25% by weight.

3. Composition according to claim 1, containing, in a physiologically acceptable aqueous medium, at least:
1) a sulfonate mixture comprising:
- one or more compounds of formula (A1): 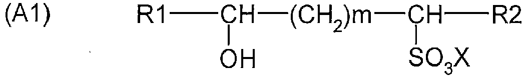 in which:
- R1 and R2 represent, independently, a hydrogen atom or a branched or linear C₁-C₁₄ alkyl radical;
- m is an integer from 0 to 14,
R1, R2 and m are such that the said compound(s) (A1) comprise 16 carbon atoms and
- X represents a cation or a mixture of cations allowing achievement of electroneutrality of the said compound(s) (A1);
- one or more compounds of formula (B1); in which: 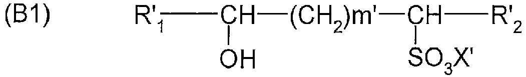
- R'1 and R'2 represent, independently, a hydrogen atom or a branched or linear C₁-C₁₆ alkyl radical;
- m' is an integer from 0 to 16,
R'1, R'2 and m' being such that the said compound(s) (B1) comprise 18 carbon atoms and
- X' represents a cation or a mixture of cations allowing achievement of electroneutrality of the said compound(s) (B1); and
- optionally one or more compounds of formula (A2) and/or (A3): 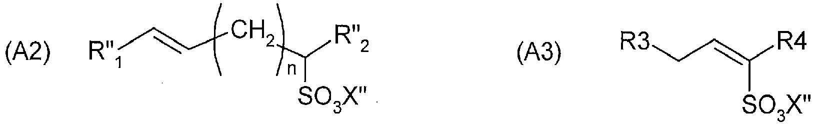 in which:
- R"1, R"2, R3 and R4 represent, independently, a hydrogen atom or a branched or linear C1-C13 alkyl radical;
- n is an integer from 0 to 13,
R"1, R"2, n, R3 and R4 being such that the said compound(s) (A2) and (A3) comprise 16 carbon atoms, and
- X" represents a cation or a mixture of cations allowing achievement of electroneutrality of the said compound(s) (A2) and/or (A3); and
- optionally one or more compounds of formula (B2) and/or (B3): 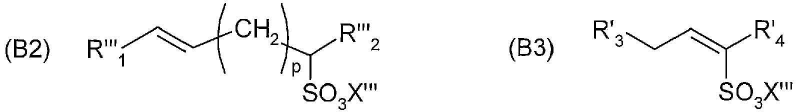 in which:
- R'''1, R'''2, R'3 and R'4 represent, independently, a hydrogen atom or a branched or linear C1-C15 alkyl radical;
- p is an integer from 0 to 15,
R'''1, R'''2, p, R'3 and R'4 being such that the said compound(s) (B2) and (B3) comprise 18 carbon atoms, and
- X''' represents a cation or a mixture of cations allowing achievement of electroneutrality of the said compound(s) (B2) and/or (B3);
wherein:
- the weight ratio of all the compounds (A1), (A2) and (A3) to all the compounds (B1), (B2) and (B3) (which means (A1+A2+A3)/(B1+B2+B3)) is from 75/25 to 90/10, preferably from 80/20 to 85/15, and
- the weight ratio of all the compounds (A1) and (B1) to all the compounds (A2), (B2), (A3) and (B3) (which means (A1+B1)/(A2+A3+B2+B3)) is from 75/25 to 100/0, preferably from 80/20 to 95/5;
2) at least one polymeric suspension agent.

4. Composition according to claim 3, wherein the sulfonate mixture comprises the compounds having a sulfonate group located on the carbon atom at C2 position, in particular the compounds (A1), (B1) and optionally the compounds (A2), (B2), (A3) and (B3) in a total amount of 28% by weight or less relative to the total weight of the sulfonate mixture, more preferably, from 10 to 28% by weight, and better from 15 to 25% by weight.

5. Composition according to claim 3 or 4, wherein the sulfonate mixture comprises
- the compound(s) (A1) in an amount of from 50 to 90% by weight, preferably from 55 to 85% by weight, more preferably from 60 to 80% by weight, relative to the total weight of the sulfonate mixture and/or
- the compound(s) (B1) in an amount of from 5 to 25% by weight, preferably from 10 to 23% by weight, more preferably from 15 to 20% by weight, relative to the total weight of the sulfonate mixture and/or
- the compound(s) (A2) and (A3) in a total amount of from 0 to 20% by weight, preferably from 1 to 20% by weight, more preferably from 5 to 15% by weight, relative to the total weight of the sulfonate mixture and/or
- the compound(s) (B2) and (B3) in a total amount of from 0 to 7% by weight, preferably from 0.5 to 5% by weight, more preferably from 1 to 4% by weight, relative to the total weight of the sulfonate mixture.

6. Composition according to claim 1 or 2, containing an aqueous physiologically acceptable medium and preferably an aqueous cosmetically acceptable medium.

7. Composition according to any one of claims 1 to 6, wherein the sulfonate mixture is present in an amount from 0.5% to 20% by weight, more preferably from 1% to 15% by weight, more particularily from 1% to 10% by weight relative to the total weight of the composition.

8. Composition according to any one of claims 1 to 7, wherein
the crosslinked homopolymers and crosslinked copolymers of acrylic acid and/or methacrylic acid and/or their esters are selected from:
(1) the homopolymers of acrylic acid crosslinked with an allyl ether of pentaerythritol, an allyl ether of sucrose, or an allyl ether of propylene;
(2) the non-associative crosslinked copolymers of acrylic acid and/or methacrylic acid and esters thereof; and
(3) the associative anionic crosslinked polymers of acrylic acid and/or methacrylic acid and/or their esters.

9. Composition according to any one of claims 1 to 8, wherein the polymeric suspending agent is present preferably from 0.1% to 10% by weight relative to the total weight of the composition, more preferably from 1 to 8 % by weight, and more particularily from 2 to 5 % by weight relative to the total weight of the composition.

10. Composition according to any one of claims 1 to 9, **characterized in that** it is soap-free and/or sulfate surfactant-free.

11. Composition according to any one of claims 1 to 10, which further contains at least one non-ionic surfactant, preferably chosen from oxyalkylenated glycerol esters, oxyalkylenated sugar esters, alkyl polyglucosides and mixtures thereof; and more preferably chosen from alkyl polyglucosides and more particularily is Caprylyl/capryl glucoside.

12. Composition according to any one of claims 1 to 11, which further contains at least one amphoteric or zwitterionic surfactant, preferably chosen from betaines, N-alkylamidobetaines and derivatives thereof, sultaines, alkyl polyaminocarboxylates, alkylamphoacetates, and mixtures thereof, and more preferably betaines and especially alkylbetaines and more particularily Coco betaine.

13. Composition according to any one of claims 1 to 12, which further contains at least one foam-enhancing agent or one foam booster, preferably selected from:
- the fatty alcohols;
- the polyalkyleneglycols;
- the polyglyceryl fatty acid esters;
- the polyalkyleneglycol ethers of alkylglucose;
- the celluloses;
- the crosslinked polymers formed by the reaction of an C₁₀-C₁₈ alkylglucoside with 1,3-dichloro-2-propanol, and sulfonated with 3-chloro-2-hydroxypropyl sulfonate;
- the fatty acid alkanolamides;
- and their mixtures
and more preferably selected from the polylethyleneglycols and polyglyceryl fatty acid esters and more particularily selected from PEG-45M and POLYGLYCERYL-2 LAURATE.

14. Composition according to any one of claims 1 to 13, which contains, in an aqueous medium:
a) at least one sulfonate mixture as defined in the preceding claims; and
b) at least one polymeric suspension as defined in the preceding claims; and
c) at least one non-ionic surfactant as defined in the preceding claims; and
d) at least one amphoteric or zwitterionic surfactant as defined in the preceding claims; and
e) at least one enhancing-foam agent or foam booster as defined in the preceding claims.

15. Composition according to claim 14, which contains:
a) at least one sulfonate mixture in an amount of from 0.5% to 20% by weight relative to the total weight, preferably from 1% to 15% by weight, more preferably from 1% to 10% by weight; and
b) at least one polymeric suspension in an amount of 0.1% to 10% by weight relative to the total weight of the composition, preferably from 1% to 8% by weight, more preferably from 2 % to 5% by weight, and
c) at least one non-ionic surfactant and preferably one alkylpolyglucoside surfactant in an amount from 0.1% to 20% by weight relative to the total weight of the composition, preferably from 0.5% to 15% by weight, more preferably from 1% to 8% by weight, and
d) at least one amphoteric or zwitterionic surfactant in an amount of from 0.1% to 20% by weight relative to the total weight of the composition, preferably from 0.15% to 15% by weight, more preferably from 1% to 10% by weight, and
e) at least one enhancing-foam agent or foam booster in an amount of from 0.05% to 10% by weight relative to the total weight of the composition, preferably from 0.1% to 5% by weight, more preferably from 0.2% to 3% by weight.

16. Composition according to any one of claims 1 to 15, wherein the pH thereof is from 3.0 to 7.0 and preferably from 3.0 to 6.5.

17. Process for cleansing keratin materials, which consists of applying to the said keratin materials a composition according to any one of claims 1 to 16, in working the said composition into a foam and then rinsing off the said composition, especially with water.

## Patentansprüche

1. Zusammensetzung, die in einem wässrigen Medium mindestens Folgendes enthält:
1) eine Sulfonatmischung, die Folgendes enthält:
a) ein internes Olefinsulfonat (A) mit 16 Kohlenstoffatomen und
b) ein internes Olefinsulfonat (B) mit 18 Kohlenstoffatomen,
- wobei das Gewichtsverhältnis (A/B) der Komponente (A) zu der Komponente (B) in der Sulfonatmischung von 75/25 bis 90/10, vorzugsweise von 80/20 bis 85/15 beträgt, und
- wobei das Gewichtsverhältnis der als Hydroxyl vorliegenden Verbindungen in den internen Olefinsulfonaten (A) und (B) zu den als Olefin vorliegenden Verbindungen in den internen Olefinsulfonaten (A) und (B) von 75/25 bis 100/0, vorzugsweise von 80/20 bis 95/5 beträgt; und
2) mindestens ein polymeres Suspensionsmittel, das ausgewählt ist aus:
- Stärken, Carrageen, Xanthangummi, Guargummi und Cellulosen wie zum Beispiel Hydroxyethylcellulose und Hydroxypropylcellulose sowie Mischungen davon;
- den nichtionischen assoziativen Polyetherurethanen;
- den vernetzten Homopolymeren und vernetzten Copolymeren von Acrylsäure und/oder Methacrylsäure und/oder ihren Estern.

2. Zusammensetzung nach Anspruch 1, wobei die Sulfonatmischung die internen Olefinsulfonate (A) und (B) mit einer in C2-Stellung an dem Kohlenstoffatom befindlichen Sulfonatgruppe in einer Gesamtmenge von 28 Gew.-% oder weniger bezogen auf das Gesamtgewicht der Sulfonatmischung, und vorzugsweise von 10 bis 28 Gew.-%, noch besser von 15 bis 25 Gew.-%, umfasst.

3. Zusammensetzung nach Anspruch 1, die in einem physiologisch akzeptablen wässrigen Medium mindestens Folgendes enthält:
1) eine Sulfonatmischung, die Folgendes umfasst:
- eine oder mehrere Verbindungen der Formel (A1): 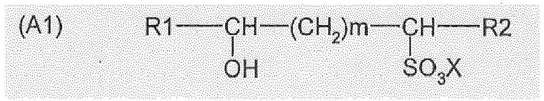 in der:
- R1 und R2 unabhängig voneinander ein Wasserstoffatom oder ein verzweigtes oder lineares C₁-C₁₄-Alkylradikal repräsentieren;
- m eine ganze Zahl von 0 bis 14 ist,
- R1, R2 und m so sind, dass die Verbindung(en) (A1) 16 Kohlenstoffatome umfasst, und
- X ein Kation oder eine Mischung von Kationen repräsentiert, mit denen Elektroneutralität der Verbindung(en) (A1) erreicht werden kann;
- eine oder mehrere Verbindungen der Formel (B1): 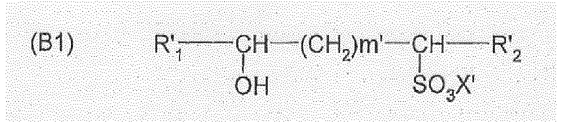 in der:
- R'1 und R'2 unabhängig voneinander ein Wasserstoffatom oder ein verzweigtes oder lineares C₁-C₁₆-Alkylradikal repräsentieren;
- m' eine ganze Zahl von 0 bis 16 ist,
wobei R'1, R'2 und m' so sind, dass die Verbindung(en) (B1) 18 Kohlenstoffatome umfassen, und
- X' ein Kation oder eine Mischung von Kationen repräsentiert, mit denen Elektroneutralität der Verbindung(en) (B1) erreicht werden kann; und
- optional eine oder mehrere Verbindungen der Formel (A2) und/oder (A3): 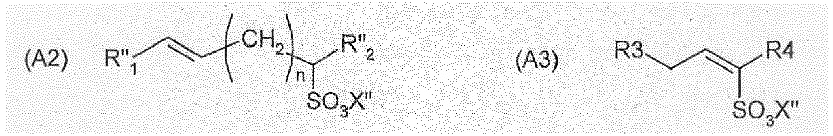 in der:
- R"1, R"2, R3 und R4 unabhängig voneinander ein Wasserstoffatom oder ein verzweigtes oder lineares C₁-C₁₃-Alkylradikal repräsentieren;
- n eine ganze Zahl von 0 bis 13 ist,
wobei R"1, R"2, n, R3 und R4 so sind, dass die Verbindung(en) (A2) und (A3) 16 Kohlenstoffatome umfassen, und
- X" ein Kation oder eine Mischung von Kationen repräsentiert, mit denen Elektroneutralität der Verbindung(en) (A2) und/oder (A3) erreicht werden kann; und
- optional eine oder mehrere Verbindungen der Formel (B2) und/oder (B3): 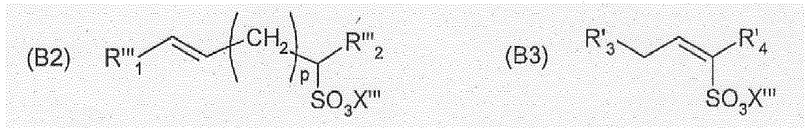 in der:
- R'''1, R'''2, R'3 und R'4 unabhängig voneinander ein Wasserstoffatom oder ein verzweigtes oder lineares C₁-C₁₅-Alkylradikal repräsentieren;
- p eine ganze Zahl von 0 bis 15 ist,
wobei R'''1, R'''2, p, R'3 und R'4 so sind, dass die Verbindung(en) (B2) und (B3) 18 Kohlenstoffatome umfassen, und
- X''' ein Kation oder eine Mischung von Kationen umfasst, mit denen Elektroneutralität der Verbindung(en) (B2) und/oder (B3) erreicht werden kann;
wobei:
- das Gewichtsverhältnis aller Verbindungen (A1), (A2) und (A3) zu allen Verbindungen (B1), (B2) und (B3) (d.h. (A1+A2+A3)/(B1+B2+B3)) von 75/25 bis 90/10, vorzugsweise von 80/20 bis 85/15 beträgt, und
- das Gewichtsverhältnis aller Verbindungen (A1) und (B1) zu allen Verbindungen (A2), (B2), (A3) und (B3) (d.h. (A1+B1)/(A2+A3+B2+B3)) von 75/25 bis 100/0, vorzugsweise von 80/20 bis 95/5 beträgt;
2) mindestens ein polymeres Suspensionsmittel.

4. Zusammensetzung nach Anspruch 3, wobei die Sulfonatmischung die Verbindungen mit einer in C2-Stellung an dem Kohlenstoffatom befindlichen Sulfonatgruppe, insbesondere die Verbindungen (A1), (B1) und optional die Verbindungen (A2), (B2), (A3) und (B3) in einer Gesamtmenge von 28 Gew.-% oder weniger bezogen auf das Gesamtgewicht der Sulfonatmischung, mehr bevorzugt von 10 bis 28 Gew.-%, besser noch von 15 bis 25 Gew.-% umfasst.

5. Zusammensetzung nach Anspruch 3 oder 4, wobei die Sulfonatmischung Folgendes umfasst:
- die Verbindung(en) (A1) in einer Menge von 50 bis 90 Gew.-%, vorzugsweise von 55 bis 85 Gew.-%, mehr bevorzugt von 60 bis 80 Gew.-%, bezogen auf das Gesamtgewicht der Sulfonatmischung und/oder
- die Verbindung(en) (B1) in einer Menge von 5 bis 25 Gew.-%, vorzugsweise von 10 bis 23 Gew.-%, mehr bevorzugt von 15 bis 20 Gew.-%, bezogen auf das Gesamtgewicht der Sulfonatmischung und/oder
- die Verbindung(en) (A2) und (A3) in einer Gesamtmenge von 0 bis 20 Gew.-%, vorzugsweise von 1 bis 20 Gew.-%, mehr bevorzugt von 5 bis 15 Gew.-%, bezogen auf das Gesamtgewicht der Sulfonatmischung und/oder
- die Verbindung(en) (B2) und (B3) in einer Gesamtmenge von 0 bis 7 Gew.-%, vorzugsweise von 0,5 bis 5 Gew.-%, mehr bevorzugt von 1 bis 4 Gew.-%, bezogen auf das Gesamtgewicht der Sulfonatmischung.

6. Zusammensetzung nach Anspruch 1 oder 2, die ein physiologisch akzeptables wässriges Medium und vorzugsweise ein kosmetisch akzeptables wässriges Medium enthält.

7. Zusammensetzung nach einem der Ansprüche 1 bis 6, wobei die Sulfonatmischung in einer Menge von 0,5 bis 20 Gew.-%, mehr bevorzugt von 1 bis 15 Gew.-%, ganz besonders von 1 bis 10 Gew.-% vorliegt, bezogen auf das Gesamtgewicht der Zusammensetzung.

8. Zusammensetzung nach einem der Ansprüche 1 bis 7, wobei die vernetzten Homopolymere und vernetzten Copolymere von Acrylsäure und/oder Methacrylsäure und/oder ihre Ester ausgewählt sind aus:
(1) den Homopolymeren von Acrylsäure, die mit einem Allylether von Pentaerythritol, einem Allylether von Saccharose oder einem Allylether von Propylen vernetzt sind;
(2) den nicht-assoziativen vernetzten Copolymeren von Acrylsäure und/oder Methacrylsäure und deren Estern; und
(3) den assoziativen anionischen vernetzten Polymeren von Acrylsäure und/oder Methacrylsäure und/oder ihren Estern.

9. Zusammensetzung nach einem der Ansprüche 1 bis 8, wobei das polymere Suspensionsmittel in einer Menge von vorzugsweise 0,1 bis 10 Gew.-% bezogen auf das Gesamtgewicht der Zusammensetzung, mehr bevorzugt von 1 bis 8 Gew.-% und ganz besonders von 2 bis 5 Gew.-% bezogen auf das Gesamtgewicht der Zusammensetzung vorliegt.

10. Zusammensetzung nach einem der Ansprüche 1 bis 9, **dadurch gekennzeichnet, dass** sie seifenfrei und/oder sulfattensidfrei ist.

11. Zusammensetzung nach einem der Ansprüche 1 bis 10, die ferner mindestens ein nichtionisches Tensid enthält, das vorzugsweise ausgewählt ist aus oxyalkylenierten Glycerinestern, oxyalkylenierten Zuckerestern, Alkylpolyglucosiden und Mischungen davon; und mehr bevorzugt ausgewählt ist aus Alkylpolyglucosiden und ganz besonders Caprylyl/Capryl-Glucosid ist.

12. Zusammensetzung nach einem der Ansprüche 1 bis 11, die ferner mindestens ein amphoteres oder zwitterionisches Tensid enthält, das vorzugsweise ausgewählt ist aus Betainen, N-Alkylamidobetainen und Derivaten davon, Sultainen, Alkylpolyaminocarboxylaten, Alkylamphoacetaten und Mischungen davon, und mehr bevorzugt aus Betainen und insbesondere Alkylbetainen und ganz besonders Kokosbetain.

13. Zusammensetzung nach einem der Ansprüche 1 bis 12, die ferner mindestens ein schaumverstärkendes Mittel bzw. einen Schaumverstärker enthält, der vorzugsweise ausgewählt ist aus:
- den Fettalkoholen;
- den Polyalkylenglycolen;
- den Polyglycerylfettsäureestern;
- den Polyalkylenglycolethern von Alkylglucose;
- den Cellulosen;
- den vernetzten Polymeren, die durch die Umsetzung eines C₁₀-C₁₈-Alkylglucosids mit 1,3-Dichlor-2-propanol gebildet sind und mit 3-Chlor-2-hydroxypropylsulfonat sulfoniert sind;
- den Fettsäurealkanolamiden;
- und ihren Mischungen
und mehr bevorzugt ausgewählt ist aus den Polyethylenglycolen und Polyglycerylfettsäureestern und ganz besonders ausgewählt ist aus PEG-45M und Polyglyceryl-2 Laurat.

14. Zusammensetzung nach einem der Ansprüche 1 bis 13, die in einem wässrigen Medium Folgendes enthält:
a) mindestens eine Sulfonatmischung gemäß den vorhergehenden Ansprüchen; und
b) mindestens eine polymere Suspension gemäß den vorhergehenden Ansprüchen; und
c) mindestens ein nichtionisches Tensid gemäß den vorhergehenden Ansprüchen; und
d) mindestens ein amphoteres oder zwitterionisches Tensid gemäß den vorhergehenden Ansprüchen; und
e) mindestens ein schaumverstärkendes Mittel bzw. einen Schaumverstärker gemäß den vorhergehenden Ansprüchen.

15. Zusammensetzung nach Anspruch 14, die Folgendes enthält:
a) mindestens eine Sulfonatmischung in einer Menge von 0,5 bis 20 Gew.-% bezogen auf das Gesamtgewicht, vorzugsweise von 1 bis 15 Gew.-%, mehr bevorzugt von 1 bis 10 Gew.-%; und
b) mindestens eine polymere Suspension in einer Menge von 0,1 bis 10 Gew.-% bezogen auf das Gesamtgewicht der Zusammensetzung, vorzugsweise von 1 bis 8 Gew.-%, mehr bevorzugt von 2 bis 5 Gew.-%, und
c) mindestens ein nichtionisches Tensid und vorzugsweise ein Alkylpolyglucosid-Tensid in einer Menge von 0,1 bis 20 Gew.-% bezogen auf das Gesamtgewicht der Zusammensetzung, vorzugsweise von 0,5 bis 15 Gew.-%, mehr bevorzugt von 1 bis 8 Gew.-%, und
d) mindestens ein amphoteres oder zwitterionisches Tensid in einer Menge von 0,1 bis 20 Gew.-% bezogen auf das Gesamtgewicht der Zusammensetzung, vorzugsweise von 0,15 bis 15 Gew.-%. mehr bevorzugt von 1 bis 10 Gew.-%, und
e) mindestens ein schaumverstärkendes Mittel bzw. einen Schaumverstärker in einer Menge von 0,05 bis 10 Gew.-% bezogen auf das Gesamtgewicht der Zusammensetzung, vorzugsweise von 0,1 bis 5 Gew.-%, mehr bevorzugt von 0,2 bis 3 Gew.-%.

16. Zusammensetzung nach einem der Ansprüche 1 bis 15, wobei ihr pH-Wert von 3,0 bis 7,0 und vorzugsweise von 3,0 bis 6,5 beträgt.

17. Verfahren zum Reinigen von Keratinmaterialien, das darin besteht, auf die Keratinmaterialien eine Zusammensetzung nach einem der Ansprüche 1 bis 16 aufzubringen, die Zusammensetzung in einen Schaum einzuarbeiten und dann die Zusammensetzung abzuspülen, insbesondere mit Wasser.

## Revendications

1. Composition contenant, dans un milieu aqueux, au moins :
1) un mélange de sulfonates contenant :
a) un (oléfine interne)-sulfonate (A) comportant 16 atomes de carbone,
b) et un (oléfine interne)-sulfonate (B) comportant 18 atomes de carbone,
étant entendu
- que le rapport pondéral (A/B) du composant A au composant B dans le mélange de sulfonates vaut de 75/25 à 90/10, et de préférence de 80/20 à 85/15,
- et que le rapport pondéral des composés sous forme hydroxylée présents dans les (oléfine interne)-sulfonates (A) et (B) aux composés sous forme oléfine présents dans les (oléfine interne)-sulfonates (A) et (B) vaut de 75/25 à 100/0, et de préférence de 80/20 à 95/5 ;
2) et au moins un agent de suspension de nature polymère, choisi parmi
- les amidons, carraghénane, gomme xanthane, gomme de guar, et celluloses telles que l'hydroxypropyl-cellulose et l'hydroxyéthyl-cellulose, et leurs mélanges,
- les polyéther-uréthanes associatifs non-ioniques,
- et les homopolymères réticulés et copolymères réticulés d'acide acrylique et/ou d'acide méthacrylique et/ou d'esters de ces acides.

2. Composition conforme à la revendication 1, dans laquelle le mélange de sulfonates comprend les (oléfine interne)-sulfonates (A) et (B) dont le groupe sulfonate se trouve sur l'atome de carbone placé en position C-2 en une proportion valant au total 28 % ou moins, en poids rapporté au poids total du mélange de sulfonates, et valant de préférence de 10 à 28 % en poids et mieux encore de 15 à 25 % en poids.

3. Composition conforme à la revendication 1, contenant, dans un milieu aqueux physiologiquement admissible, au moins :
1) un mélange de sulfonates comprenant :
- un ou plusieurs composé(s) de formule (A1) 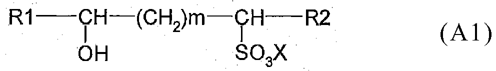 dans laquelle
- les symboles R1 et R2 représentent chacun, indépendamment, un atome d'hydrogène ou un groupe alkyle en C₁-C₁₄, linéaire ou ramifié,
- l'indice m est un nombre entier valant de 0 à 14,
étant entendu que les entités R1 et R2 et l'indice m sont tels que ledit ou lesdits composé(s) (A1) comporte(nt) 16 atomes de carbone,
- et X représente un cation ou un mélange de cations assurant l'électro-neutralité dudit ou desdits composé(s) (A1),
- et un ou plusieurs composé(s) de formule (B1) 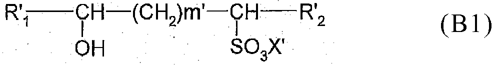 dans laquelle
- les symboles R'₁ et R'₂ représentent chacun, indépendamment, un atome d'hydrogène ou un groupe alkyle en C₁-C₁₆, linéaire ou ramifié,
- l'indice m' est un nombre entier valant de 0 à 16,
étant entendu que les entités R'₁ et R'₂ et l'indice m' sont tels que ledit ou lesdits composé(s) (B1) comporte(nt) 18 atomes de carbone,
- et X' représente un cation ou un mélange de cations assurant l'électro-neutralité dudit ou desdits composé(s) (B1),
- ainsi que, en option, un ou plusieurs composé(s) de formule(s) (A2) et/ou (A3) 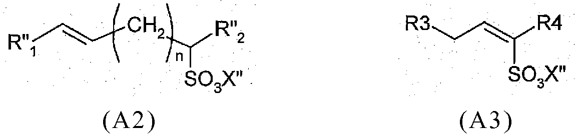
dans lesquelles
- les symboles R"₁, R"₂, R3 et R4 représentent chacun, indépendamment, un atome d'hydrogène ou un groupe alkyle en C₁-C₁₃, linéaire ou ramifié,
- l'indice n est un nombre entier valant de 0 à 13,
étant entendu que les entités R"₁, R"₂, R3 et R4 et l'indice n sont tels que ledit ou lesdits composé(s) (A2) et (A3) comportent) 16 atomes de carbone,
- et X" représente un cation ou un mélange de cations assurant l'électro-neutralité dudit ou desdits composé(s) (A2) et/ou (A3),
- et en option, un ou plusieurs composé(s) de formule(s) (B2) et/ou (B3) 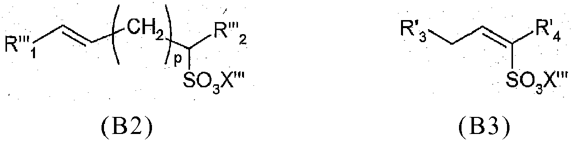 dans lesquelles
- les symboles R'''₁, R'''₂, R'₃ et R'₄ représentent chacun, indépendamment, un atome d'hydrogène ou un groupe alkyle en C₁-C₁₅, linéaire ou ramifié,
- l'indice p est un nombre entier valant de 0 à 15,
étant entendu que les entités R'''₁, R'''₂, R'₃ et R'₄ et l'indice p sont tels que ledit ou lesdits composé(s) (B2) et (B3) comporte(nt) 18 atomes de carbone,
- et X'" représente un cation ou un mélange de cations assurant l'électro-neutralité dudit ou desdits composé(s) (B2) et/ou (B3),
étant entendu
- que le rapport pondéral de tous les composés (A1), (A2) et (A3) à tous les composés (B1), (B2) et (B3), autrement dit le rapport (A1+A2+A3)/(B1+B2+B3), vaut de 75/25 à 90/10 et de préférence de 80/20 à 85/15,
- et que le rapport pondéral de tous les composés (A1) et (B1) à tous les composés (A2), (B2), (A3) et (B3), autrement dit le rapport (A1+B1)/(A2+A3+B2+B3), vaut de 75/25 à 100/0 et de préférence de 80/20 à 95/5,
2) et au moins un agent de suspension de nature polymère.

4. Composition conforme à la revendication 3, dans laquelle le mélange de sulfonates comprend les (oléfine interne)-sulfonates dont le groupe sulfonate se trouve sur l'atome de carbone placé en position C-2, en particulier les composés (A1) et (B1) ainsi que les composés optionnels (A2), (B2), (A3) et (B3), en une proportion valant au total 28 % ou moins, en poids rapporté au poids total du mélange de sulfonates, et valant de préférence de 10 à 28 % en poids et mieux encore de 15 à 25 % en poids.

5. Composition conforme à la revendication 3 ou 4, dans laquelle le mélange de sulfonates comprend
- le ou les composé(s) (A1) en une proportion valant de 50 à 90 % en poids, de préférence de 55 à 85 % en poids et mieux encore de 60 à 80 % en poids rapporté au poids total du mélange de sulfonates,
- et/ou le ou les composé(s) (B1) en une proportion valant de 5 à 25 % en poids, de préférence de 10 à 23 % en poids et mieux encore de 15 à 20 % en poids rapporté au poids total du mélange de sulfonates,
- et/ou le ou les composé(s) (A2) et A3) en une proportion valant au total de 0 à 20 % en poids, de préférence de 1 à 20 % en poids et mieux encore de 5 à 15 % en poids rapporté au poids total du mélange de sulfonates,
- et/ou le ou les composé(s) (B2) et (B3) en une proportion valant au total de 0 à 7 % en poids, de préférence de 0,5 à 5 % en poids et mieux encore de 1 à 4 % en poids rapporté au poids total du mélange de sulfonates.

6. Composition conforme à la revendication 1 ou 2, contenant un milieu aqueux physiologiquement admissible et de préférence un milieu aqueux cosmétologiquement admissible.

7. Composition conforme à l'une des revendications 1 à 6, dans laquelle le mélange de sulfonates se trouve présent en une proportion valant de 0,5 à 20 % en poids, de préférence de 1 à 15 % en poids et mieux encore de 1 à 10 % en poids rapporté au poids total de la composition.

8. Composition conforme à l'une des revendications 1 à 7, dans laquelle les homopolymères réticulés et copolymères réticulés d'acide acrylique et/ou d'acide méthacrylique et/ou d'esters de ces acides sont choisis parmi :
1) les homopolymères d'acide acrylique, réticulés avec un éther allylique de pentaérythritol, un éther allylique de saccharose ou l'éther allylique de propylène,
2) les copolymères réticulés non-associatifs d'acide acrylique et/ou d'acide méthacrylique et/ou d'esters de ces acides,
3) et les copolymères réticulés anioniques associatifs d'acide acrylique et/ou d'acide méthacrylique et/ou d'esters de ces acides.

9. Composition conforme à l'une des revendications 1 à 8, dans laquelle l'agent de suspension de nature polymère se trouve présent en une proportion valant de préférence de 0,1 à 10 % en poids rapporté au poids total de la composition, mieux encore de 1 à 8 % en poids, et plus particulièrement de 2 à 5 % en poids rapporté au poids total de la composition.

10. Composition conforme à l'une des revendications 1 à 9, **caractérisée en ce qu'**elle ne contient ni savon, ni tensioactif de type sulfate.

11. Composition conforme à l'une des revendications 1 à 10, qui contient en outre au moins un tensioactif non-ionique, de préférence choisi parmi les esters de glycérol alcoxylés, les esters de sucre alcoxylés, les alkyl-polyglucosides, et leurs mélanges, et mieux encore, choisi parmi les alkylpolyglucosides, et plus particulièrement, qui est un caprylyl/capryl-glucoside.

12. Composition conforme à l'une des revendications 1 à 11, qui contient en outre au moins un tensioactif amphotère ou zwitterionique, choisi de préférence parmi les bétaïnes, les N-alkyl-amido-bétaïnes et leurs dérivés, les sultaïnes, les polyaminocarboxylates d'alkyle, les amphoacétates d'alkyle, et leurs mélanges, et mieux encore parmi les bétaïnes et spécialement les alkyl-bétaïnes, et qui est plus particulièrement la coco-bétaïne.

13. Composition conforme à l'une des revendications 1 à 12, qui contient en outre au moins un agent intensifiant le moussage ou un agent accélérant le moussage, choisi de préférence parmi
- les alcools gras,
- les polyalkylène-glycols,
- les esters de polyglycéryle et d'acide gras,
- les éthers de polyalkylène-glycol et d'alkyl-glucose,
- les celluloses,
- les polymères réticulés formés par réaction d'un (alkyle en C₁₀-C₁₈)-glucoside et du 1,3-dichloro-propan-2-ol et sulfonés au moyen de 3-chloro-2-hydroxy-propane-sulfonate,
- les alcanolamides d'acide gras,
- et les mélanges de tels composés,
et mieux encore, choisi parmi les polyéthylèneglycols et les esters de polyglycéryle et d'acide gras, et plus particulièrement, choisi parmi le PEG-45M et le laurate de polyglycéryle-2.

14. Composition conforme à l'une des revendications 1 à 13, qui contient, dans un milieu aqueux :
a) au moins un mélange de sulfonates, tel que défini dans les revendications précédentes,
b) au moins un agent de suspension de nature polymère, tel que défini dans les revendications précédentes,
c) au moins un tensioactif non-ionique, tel que défini dans les revendications précédentes,
d) au moins un tensioactif amphotère ou zwitterionique, tel que défini dans les revendications précédentes,
e) et au moins un agent intensifiant le moussage ou un agent accélérant le moussage, tel que défini dans les revendications précédentes.

15. Composition conforme à la revendication 14, qui contient :
a) au moins un mélange de sulfonates, en une proportion valant de 0,5 à 20 % en poids rapporté au poids total de la composition, de préférence de 1 à 15 % en poids, et mieux encore de 1 à 10 % en poids,
b) au moins un agent de suspension de nature polymère, en une proportion valant de 0,1 à 10 % en poids rapporté au poids total de la composition, de préférence de 1 à 8 % en poids, et mieux encore de 2 à 5 % en poids,
c) au moins un tensioactif non-ionique, et de préférence un tensioactif de type alkyl-polyglucoside, en une proportion valant de 0,1 à 20 % en poids rapporté au poids total de la composition, de préférence de 0,5 à 15 % en poids, et mieux encore de 1 à 8 % en poids,
d) au moins un tensioactif amphotère ou zwitterionique, en une proportion valant de 0,1 à 20 % en poids rapporté au poids total de la composition, de préférence de 0,15 à 15 % en poids, et mieux encore de 1 à 10 % en poids,
e) et au moins un agent intensifiant le moussage ou un agent accélérant le moussage, en une proportion valant de 0,05 à 10 % en poids rapporté au poids total de la composition, de préférence de 0,1 à 5 % en poids, et mieux encore de 0,2 à 3 % en poids.

16. Composition conforme à l'une des revendications 1 à 15, dont le pH vaut de 3,0 à 7,0, et de préférence de 3,0 à 6,5.

17. Procédé de nettoyage de matières kératiniques, qui consiste à appliquer sur lesdites matières kératiniques une composition conforme à l'une des revendications 1 à 16, à travailler cette composition de manière à ce qu'elle forme une mousse, et à enlever ensuite cette composition par rinçage, en particulier avec de l'eau.
